# EUROPEAN PATENT SPECIFICATION

(11) **EP 2 813 243 B1**
(45) Date of publication and mention of the grant of the patent: **09.08.2017**
(21) Application number: 13171445.3
(22) Date of filing: 11.06.2013
(51) Int. Cl.: A61K 39/35, C07K 14/415

(54) **PRU p 3 mimicks**
PRU p 3-Mimiken
Imitations de PRU p 3

(43) Date of publication of application: 17.12.2014
(73) Proprietor: Paris-Lodron-Universität Salzburg, 5020 Salzburg (AT)
(72) Inventor: Gadermaier, Gabriele, 5300 Hallwang (AT); Ferreira, Fatima, 5020 Salzburg (AT); Lackner, Peter, 5020 Salzburg (AT); Wallner, Michael, 4600 Wels (AT)
(74) Representative: Gassner, Birgitta

(56) References cited:
- WO-A1-02/20790
- LAURIAN ZUIDMEER-JONGEJAN ET AL: "FAST: towards safe and effective subcutaneous immunotherapy of persistent life-threatening food allergies", CLINICAL AND TRANSLATIONAL ALLERGY, BIOMED CENTRAL LTD, LONDON, UK, vol. 2, no. 1, 9 March 2012 (2012-03-09), page 5, XP021096095, ISSN: 2045-7022, DOI: 10.1186/2045-7022-2-5
- MASAKO TODA ET AL: "Protein unfolding strongly modulates the allergenicity and immunogenicity of Pru p 3, the major peach allergen", JOURNAL OF ALLERGY AND CLINICAL IMMUNOLOGY, vol. 128, no. 5, 1 November 2011 (2011-11-01), pages 1022-1030.e7, XP055023729, ISSN: 0091-6749, DOI: 10.1016/j.jaci.2011.04.020
- G GARCÍA-CASADO: "Identification of IgE-binding epitopes of the major peach allergen Pru p 3", JOURNAL OF ALLERGY AND CLINICAL IMMUNOLOGY, vol. 112, no. 3, 1 September 2003 (2003-09-01), pages 599-605, XP055023735, ISSN: 0091-6749, DOI: 10.1016/S0091-6749(03)01605-1

### SEQUENCE LISTING

<110> Paris Lodron Universität Salzburg
<120> PRU p 3 MIMICKS
<130> US001EP
<160> 10
<170> PatentIn version 3.5
<210> 1
   <211> 91
   <212> PRT
   <213> Artificial Sequence
<220>
   <223> Pru p 3 wild-typ
<400> 1
   Ile Thr Cys Gly Gln Val Ser Ser Ser Leu Ala Pro Cys Ile Pro Tyr
   1               5                   10                  15
   Val Arg Gly Gly Gly Ala Val Pro Pro Ala Cys Cys Asn Gly Ile Arg
               20                  25                  30
   Asn Val Asn Asn Leu Ala Arg Thr Thr Pro Asp Arg Gln Ala Ala Cys
           35                  40                  45
   Asn Cys Leu Lys Gln Leu Ser Ala Ser Val Pro Gly Val Asn Pro Asn
       50                  55                  60
   Asn Ala Ala Ala Leu Pro Gly Lys Cys Gly Val Ser Ile Pro Tyr Lys
   65                  70                  75                  80
   Ile Ser Ala Ser Thr Asn Cys Ala Thr Val Lys
                   85                  90
<210> 2
   <211> 13
   <212> PRT
   <213> Artificial Sequence
<220>
   <223> Pru p 3 T-cell epitope
<400> 2
   Cys Ile Pro Tyr Val Arg Gly Gly Gly Ala Val Pro Pro
   1               5                   10
<210> 3
   <211> 15
   <212> PRT
   <213> Artificial Sequence
<220>
   <223> Pru p 3 T-cell epitope
<400> 3
   Asn Asn Leu Ala Arg Thr Thr Pro Asp Arg Gln Ala Ala Cys Asn
   1               5                   10                  15
<210> 4
   <211> 14
   <212> PRT
   <213> Artificial Sequence
<220>
   <223> Pru p 3 T-cell epitope
<400> 4
   Asp Arg Gln Ala Ala Cys Asn Cys Leu Lys Gln Leu Ser Ala
   1 5 10
<210> 5
   <211> 13
   <212> PRT
   <213> Artificial Sequence
<220>
   <223> Pru p 3 T-cell epitope
<400> 5
   Val Asn Pro Asn Asn Ala Ala Ala Leu Pro Gly Lys Cys
   1 5 10
<210> 6
   <211> 92
   <212> PRT
   <213> Artificial Sequence
<220>
   <223> mutant Pru p 3
<220>
   <221> misc_feature
   <222> (1)..(1)
   <223> M = methionine or no amino acid residue
<220>
   <221> misc_feature
   <222> (4)..(4)
   <223> X = any amino acid residue
<220>
   <221> misc_feature
   <222> (7)..(7)
   <223> X = valine or proline
<220>
   <221> misc_feature
   <222> (11)..(11)
   <223> X = leucine or proline
<220>
   <221> misc_feature
   <222> (14)..(14)
   <223> X = any amino acid residue
<220>
   <221> misc_feature
   <222> (28) .. (28)
   <223> X = cysteine, proline or argenine
<220>
   <221> misc_feature
   <222> (29) .. (29)
   <223> X = cysteine, proline or argenine
<220>
   <221> misc_feature
   <222> (32)..(32)
   <223> X = isoleucine or proline
<220>
   <221> misc_feature
   <222> (35)..(35)
   <223> X = valine or proline
<220>
   <221> misc_feature
   <222> (49) .. (49)
   <223> X = any amino acid residue
<220>
   <221> misc_feature
   <222> (51)..(51)
   <223> X = any amino acid residue
<220>
   <221> misc_feature
   <222> (74)..(74)
   <223> X = any amino acid residue
<220>
   <221> misc_feature
   <222> (88)..(88)
   <223> X = any amino acid residue
<400> 6
   Met Ile Thr Xaa Gly Gln Xaa Ser Ser Ser Xaa Ala Pro Xaa Ile Pro
   1               5                   10                  15
   Tyr Val Arg Gly Gly Gly Ala Val Pro Pro Ala Xaa Xaa Asn Gly Xaa
               20                  25                  30
   Arg Asn Xaa Asn Asn Leu Ala Arg Thr Thr Pro Asp Arg Gln Ala Ala
           35                  40                  45
   Xaa Asn Xaa Leu Lys Gln Leu Ser Ala Ser Val Pro Gly Val Asn Pro
       50                  55                  60
   Asn Asn Ala Ala Ala Leu Pro Gly Lys Xaa Gly Val Ser Ile Pro Tyr
   65                  70                  75                  80
   Lys Ile Ser Ala Ser Thr Asn Xaa Ala Thr Val Lys
                   85                  90
<210> 7
   <211> 91
   <212> PRT
   <213> Artificial Sequence
<220>
   <223> construct 1
<400> 7
   Ile Thr Cys Gly Gln Pro Ser Ser Ser Pro Ala Pro Cys Ile Pro Tyr
   1               5                   10                  15
   Val Arg Gly Gly Gly Ala Val Pro Pro Ala Cys Cys Asn Gly Pro Arg
               20                  25                  30
   Asn Pro Asn Asn Leu Ala Arg Thr Thr Pro Asp Arg Gln Ala Ala Cys
           35                  40                  45
   Asn Cys Leu Lys Gln Leu Ser Ala Ser Val Pro Gly Val Asn Pro Asn
       50                  55                  60
   Asn Ala Ala Ala Leu Pro Gly Lys Cys Gly Val Ser Ile Pro Tyr Lys
   65                  70                  75                  80
   Ile Ser Ala Ser Thr Asn Cys Ala Thr Val Lys
                   85                  90
<210> 8
   <211> 92
   <212> PRT
   <213> Artificial Sequence
<220>
   <223> construct 1
<400> 8
   Met Ile Thr Cys Gly Gln Pro Ser Ser Ser Pro Ala Pro Cys Ile Pro
   1               5                   10                  15
   Tyr Val Arg Gly Gly Gly Ala Val Pro Pro Ala Cys Cys Asn Gly Pro
               20                  25                  30
   Arg Asn Pro Asn Asn Leu Ala Arg Thr Thr Pro Asp Arg Gln Ala Ala
           35                  40                  45
   Cys Asn Cys Leu Lys Gln Leu Ser Ala Ser Val Pro Gly Val Asn Pro
       50                  55                  60
   Asn Asn Ala Ala Ala Leu Pro Gly Lys Cys Gly Val Ser Ile Pro Tyr
   65                  70                  75                  80
   Lys Ile Ser Ala Ser Thr Asn Cys Ala Thr Val Lys
                   85                  90
<210> 9
   <211> 91
   <212> PRT
   <213> Artificial Sequence
<220>
   <223> construct 4
<400> 9
   Ile Thr Cys Gly Gln Val Ser Ser Ser Leu Ala Pro Cys Ile Pro Tyr
   1               5                   10                  15
   Val Arg Gly Gly Gly Ala Val Pro Pro Ala Ala Ala Asn Gly Ile Arg
               20                  25                  30
   Asn Val Asn Asn Leu Ala Arg Thr Thr Pro Asp Arg Gln Ala Ala Cys
           35                  40                  45
   Asn Cys Leu Lys Gln Leu Ser Ala Ser Val Pro Gly Val Asn Pro Asn
       50                  55                  60
   Asn Ala Ala Ala Leu Pro Gly Lys Cys Gly Val Ser Ile Pro Tyr Lys
   65                  70                  75                  80
   Ile Ser Ala Ser Thr Asn Cys Ala Thr Val Lys
                   85                  90
<210> 10
   <211> 102
   <212> PRT
   <213> Artificial Sequence
<220>
   <223> construct 4
<400> 10
   Met Ala Ile Thr Cys Gly Gln Val Ser Ser Ser Leu Ala Pro Cys Ile
   1               5                    10                   15
   Pro Tyr Val Arg Gly Gly Gly Ala Val Pro Pro Ala Ala Ala Asn Gly
               20                   25                   30
   Ile Arg Asn Val Asn Asn Leu Ala Arg Thr Thr Pro Asp Arg Gln Ala
           35                   40                   45
   Ala Cys Asn Cys Leu Lys Gln Leu Ser Ala Ser Val Pro Gly Val Asn
       50                   55                   60
   Pro Asn Asn Ala Ala Ala Leu Pro Gly Lys Cys Gly Val Ser Ile Pro
   65                  70                   75                   80
   Tyr Lys Ile Ser Ala Ser Thr Asn Cys Ala Thr Val Lys Cys Gly Arg
                   85                   90                   95
   Ala Thr Thr Val Lys Cys
               100

## Description

The present invention relates to recombinant Pru p 3 mimicks having the sequence of SEQ ID NO:1, which are modified by substituting at least one amino acid and which are capable of inducing IgG antibodies that are cross-reactive to the Pru p 3 wild-type allergen, and wherein reactivity to specific IgE antibodies is reduced.

### BACKGROUND

Allergic diseases are caused by an abnormality of the immune system and are caused by the production of particular antibodies of the IgE class, specific toward ubiquitary substances (allergens).

Food allergies are strongly increasing whereby the only therapy against these forms of allergy appeared to be represented by the elimination of the suspected food from the diet. Allergic reactions to peach are widespread in the Mediterranean area and especially risky because they can cause, among other, serious systemic symptoms and anaphylaxis. Up to 60% of all allergics which react with fruits and/or vegetables are allergic to peach. The major allergen is Pru p 3, which belongs to the family of non-specific lipid transfer proteins. Pru p 3 is stabilized through compact disulfide-structure and particularly resistant to both heat and gastric digestion. Because of the potentially serious symptoms patients often avoid many other foods that contain homologous allergens, which in turn lead to a restriction of the diet and thus a decrease on the quality of life. In Spain 13% and in Italy 9.8% of allergic are sensitized against Pur p 3. Up to present allergen-specific immunotherapy for food allergies, including peach allergy has not been established. No treatment of food allergies based on the use of food extracts with wild-type allergens is currently available for standard treatment because of sometimes severe side effects caused by wild-type allergens.

Use of allergens with reduced allergenic potential and preserved immunogenicity could improve the safety and efficacy of allergen-specific immunotherapy. Toda et al. (J Allergy Clin Immunol. 2011 Nov;128(5):1022-30) describe the chemical treatment of the major allergen Pru p 3 to unfold the protein which leads to decreased IgE reactivity.

EP2320946 describes allergoids derived from allergens by chemical functionalization in order to reduce the risk to induce side-effects when employed as antiallergic vaccine in the immunotherapy of the allergic diseases.

Due to structural impairment, chemical modified allergens (allergoids) may not be appropriate as a potential immunotherapy due to difficulties in standardization.

Classical allergen-specific immunotherapy, using subcutaneous injections with aqueous food extracts may be effective but has proven to be accompanied by too many anaphylactic side-effects.

Zuidmeer-Jongejan et al. propose two different strategies to produce hypoallergenic lipid transfer proteins (LTP) for safe treatment of fruit allergy (Clin Transl Allergy. 2012 Mar 9;2(1):5).

The development of a hypoallergenic therapeutic agent against Pru p 3-mediated peach allergy is therefore important because i) peach allergy is clinically relevant and patients are faced with severe symptoms, ii) it is a food allergy, which persists as a rule for life, iii) except avoidance and rescue medication no form of treatment is currently available, and iv) less side effects are to be expected due to the alteration of the molecule.

### SHORT DESCRIPTION OF THE INVENTION

The selective exchange of individual amino acids leads to a partial structural alteration of the Pru p 3 allergen. Thus, the present invention relates to recombinant Pru p 3 mimicks having the sequence of SEQ ID NO:1, which are modified by substituting at least one amino acid and which are capable of inducing IgG antibodies that are cross-reactive to the Pru p 3 wild-type allergen, and where reactivity to specific IgE antibodies is reduced, wherein at least 1, 2, 3, 4, 5, or 6 amino acids are substituted at any position outside the T-cell epitopes.

### DETAILED DESCRIPTION OF THE INVENTION

The present invention relates therefore to a recombinant Pru p 3 mimick having the sequence of SEQ ID NO:1, which is modified by substituting at least one amino acid and which is capable of inducing IgG antibodies that are cross-reactive to the Pru p 3 wild-type allergen, and wherein reactivity to specific IgE antibodies is reduced, wherein at least 1, 2, 3, 4, 5, or 6 amino acids are substituted at any position outside the T-cell epitopes. "Cross-reactive" is used in its broadest sense and refers generally to a protein capable of detectable binding to an antibody, the latter being specific to Pru p 3 or to derivatives or homologues of Pru p 3. Such an immunologically related allergen is referred to herein as an immunological relative of Pru p 3.

The Pru p 3 mimick may be able to stimulate Pru p 3 specific T cells. This fact can be expected since the T cell epitopes in the primary sequence are not altered.

A further embodiment of the invention is the Pru p 3 mimick as described above, which shows an IgE reactivity reduction by at least 10% compared with the wild-type Pru p 3 allergen.

The Pru p 3 mimick according to the present invention shows an IgE reactivity reduction by at least 10% compared with the wild-type molecule, preferably by at least 25%, preferably by at least 50%, and more preferably by at least 80%, in the serum of patients allergic to peach.

A further embodiment of the invention is the Pru p 3 mimick as described above, wherein the at least one substituted amino acid is proline introduced at a position outside the T-cell epitopes.

A further embodiment of the invention is the Pru p 3 mimick as described above, wherein said at least one substituted amino acid is at least one proline at a position outside the T-cell epitopes (SEQ ID NO:2, SEQ ID NO:3, SEQ ID NO:4 and SEQ ID NO:5).

A further embodiment of the invention is the Pru p 3 mimick as described above, wherein at least one of the cysteines is substituted for another amino acid.

A further embodiment of the invention is the Pru p 3 mimick as described above, wherein the cysteines at positions 3, 48, 50 and/or 87 remain unsubstituted.

A further embodiment of the invention is the Pru p 3 mimick as described above, wherein the Pru p 3 mimick has an alpha helical secondary structure stabilized by at least the distal disulfide bonds.

A further embodiment of the invention is the Pru p 3 mimick, wherein the amino sequence is described by the general formula:
wherein X1 is any amino acid residue, preferably it is C,
X2 is a V or P residue,
X3 is a L or P residue,
X4 is any amino acid residue, preferably it is A or C,
X5 is a C, P or A residue, preferably it is A,
X6 is a C, P or A residue, preferably it is A,
X7 is a I or P residue,
X8 is a V or P residue,
X9 is any amino acid residue, preferably it is A or C,
X10 is any amino acid residue, preferably it is C,
X11 is any amino acid residue, preferably it is A or C,
X12 is any amino acid residue, preferably it is A or C,
n can be either 0 or 1, and wherein at least one of positions X1, X2, X3, X4, X5, X6, X7, X8, X9, X10, X11 or X12 are modified (SEQ ID No. 6).

A further embodiment of the invention is the Pru p 3 mimick as described above, wherein at least one of X2, X3, X7 or X8 is proline, prefereably X2, X3, X7 and X8 are proline.

A further embodiment of the invention is the Pru p 3 mimick as described above, wherein at least one of X1, X4, X5, X6, X9, X10, X11 and X12 is different from cysteine. Preferably X1, X4, X9, X10, X11 and X12 denote cysteine and X5 and X6 denote alanine.

A further embodiment of the invention is the Pru p 3 mimick of the amino sequence ITCGQPSSSPAPCIPYVRGGGAVPPACCNGPRNPNNLARTTPDRQAACN CLKQLSASVPGVNPNNAAALPGKCGVSIPYKISASTNCATVK (SEQ ID NO:7) or ITCGQVSSSLAPCIPYVRGGGAVPPAAANGIRNVNNLARTTPDRQAACNCLKQLSAS VPGVNPNNAAALPGKCGVSIPYKI SASTNCATVK (SEQ ID NO:9).

A further embodiment of the invention is a method for producing a recombinant Pru p 3 mimick as described above, using recombinant technique, wherein a nucleotide sequence encoding the Pru p 3 mimick is constructed in a vector and expressed in a host cell.

A further embodiment of the invention is an isolated nucleic acid molecule encoding for a Pru p 3 mimick protein as described above.

A further embodiment of the invention is the Pru p 3 mimick as described above for use as a medicament.

A further embodiment of the invention is the Pru p 3 mimick as described above for use as a medicament which stimulates Pru p 3 specific T cells.

A further embodiment of the invention is the use as described above, wherein said Pru p 3 mimick induces specific IgG antibodies capable of recognizing also the corresponding wild type allergen, but reduces capability of induction of specific IgE antibodies.

A further embodiment of the invention is a pharmaceutical composition comprising the Pru p 3 mimick as described above, in combination with pharmaceutically acceptable excipients for the prophylactic or therapeutic treatment of allergic diseases.

A further embodiment of the invention is a vaccine comprising the Pru p 3 mimick as described above, preferably comprising further at least one pharmaceutical acceptable adjuvant, excipient and/or carrier.

A further embodiment of the invention is the vaccine as described above formulated for oral, nasal, topical, subcutaneous, sublingual, intradermal, buccal, epidermal administration or for administration by inhalation, by injection, or by a patch.

The Pru p 3 mimicks of the invention may be provided to an individual either as single administration or in combination. Each molecule of the invention may be provided to an individual in an isolated, substantially isolated, purified or substantially purified form. For example, a Pru p 3 mimick of the invention may be provided to an individual substantially free from other peptides.

Whilst it may be possible for the Pru p 3 mimicks according to the invention to be presented in raw form, it is preferable to present them as a pharmaceutical formulation. Thus, according to a further aspect of the invention, the present invention provides a pharmaceutical formulation for use in preventing or treating allergy to peach by tolerization comprising a composition, vector or product according to the invention together with one or more pharmaceutically acceptable carriers or diluents and optionally one or more other therapeutic ingredients. The carrier(s) must be acceptable in the sense of being compatible with the other ingredients of the formulation and not deleterious to the recipient thereof. Typically, carriers for injection, and the final formulation, are sterile and pyrogen free.

Formulation of a composition comprising the Pru p 3 mimicks of the invention can be carried out using standard pharmaceutical formulation chemistries and methodologies all of which are readily available to the reasonably skilled artisan.

For example, compositions containing Pru p 3 mimicks of the invention can be combined with one or more pharmaceutically acceptable excipients or vehicles. Auxiliary substances, such as wetting or emulsifying agents, pH buffering substances and the like, may be present in the excipient or vehicle. These excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethyleneglycol, hyaluronic acid, glycerol, thioglycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like.

Such compositions may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition for parenteral administration, the active ingredient is provided in dry (for e.g., a powder or granules) form for reconstitution with a suitable vehicle (e. g., sterile pyrogen-free water) prior to parenteral administration of the reconstituted composition. The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or diglycerides.

Other parenterally-administrable compositions which are useful include those which comprise the active ingredient in macrocrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

Alternatively, the Pru p 3 mimicks of the present invention may be encapsulated, adsorbed to, or associated with, particulate carriers. Suitable particulate carriers include those derived from polymethyl methacrylate polymers, as well as PLG microparticles derived from poly(lactides) and poly(lactide-co-glycolides). Other particulate systems and polymers can also be used, for example, polymers such as polylysine, polyarginine, polyonuthine, spermine, spermidine, as well as conjugates of these molecules.

The formulation of any of the Pru p 3 mimicks mentioned herein will depend upon factors such as the nature of the substance and the method of delivery. Any such substance may be administered in a variety of dosage forms. It may be administered orally (e.g. as tablets, troches, lozenges, aqueous or oily suspensions, dispersible powders or granules), topically, parenterally, subcutaneously, by inhalation, intravenously, intramuscularly, intrasternally, transdermally, intradermally, sublingually, intranasally, buccally or by infusion techniques. The substance may also be administered as suppositories. A physician will be able to determine the required route of administration for each particular individual.

The compositions of formulations of the invention will comprise a suitable concentration of each Pru p 3 mimick to be effective without causing adverse reaction. Typically, the concentration of each Pru p 3 mimick in the composition will be in the range of 0.03 to 200 nmol/ml. More preferably in the range of 0.3 to 200 nmol/ml, 3 to 180 nmol/ml, 10 to 150 nmol/ml, 50 to 120 nmol/ml or 30 to 100 nmol/ml. The composition or formulations should have a purity of greater than 95% or 98% or a purity of at least 99%.

In one aspect of the invention an adjuvant may be used in combination with the Pru p 3 mimick of the invention. The adjuvant is preferably administered in an amount which is sufficient to augment the effect of the Pru p 3 mimick of the invention or vice versa. The adjuvant or other therapeutic agent may be an agent that potentiates the effects of the molecule of the invention. For example, the other agent may be an immunomodulatory molecule or an adjuvant which enhances the response to the peptide or cell of the invention.

The present invention relates to Pru p 3 mimicks that are capable of desensitizing or tolerising human individuals to the allergens described above and are therefore useful in the prevention or treatment of peach allergy. The invention provides compositions, products, vectors and formulations for use in preventing or treating allergy to peach. The invention also provides a method of tolerising or desensitizing a peach allergic individual comprising administering, either singly or in combination the Pru p 3 mimicks of the invention as described above.

The individual to be treated or provided with the composition or formulation of the invention is preferably human. It will be appreciated that the individual to be treated may be known to be sensitized to the allergens, at risk of being sensitized or suspected of being sensitized. The individual can be tested for sensitisation using techniques well known in the art and as described herein. Alternatively, the individual may have a family history of allergy to peach.

### SHORT DESCRIPTION OF THE FIGURES

Fig. 1 shows a model of wild type Pru p 3; residues marked in red are altered in the sequence of Pru p 3 construct 1.
Fig. 2 shows the supernatant of centrifugation after the precipitation step.
Fig. 3 shows the cation exchange load, flow through and fraction 15 - 29.
Fig. 4 shows the aliquots stored at 4°C; lyophilized and then stored at -20°C; stored in solution at -20°C.
Fig. 5 shows the CD spectra taken at pH 7.4.
Fig. 6 shows the CD spectra taken at pH 3.0.
Fig. 7 shows patient ELISA measured absorbance of WT Pru p 3 and Pru p 3 construct 1.
Fig. 8 shows the SDS-PAGE of monomeric Pru p 3 construct 1
Fig. 9 shows a model of Pru p 3 construct 4 and exchanged cysteine residues in red.
Fig. 10 shows the Pru p 3 primary sequence.
Fig. 11 shows the SDS-PAGE Pru p 3 construct 4 using the IMPACT system
Fig. 12 shows the SDS-PAGE purification of Pru p 3 construct 4
Fig. 13 shows the CD graph pH 7.4
Fig. 14 shows the CD graph pH 3.0
Fig. 15 shows patient ELISA results
Fig. 16 shows SDS-PAGE reducing/non reducing

The present invention is further illustrated by the following examples, however, without being restricted thereto.

### EXAMPLES

### Example 1: Pru p 3 construct 1

Pru p 3 construct 1 has four amino acids exchanged compared to the wild type protein sequence. Destabilizing residues were identified, and exchanged for proline (knowledge based using *https:*//***prosa**.services.came.sbg.ac.at*/***prosa**.php).* The four amino acids that were replaced by a proline are located in two alpha helical regions of the protein. As a result of the mutations the helical structure of the wild type molecule is supposed to be disrupted in Pru p 3 construct 1. The following mutations were introduced in the sequence: V6P, L10P, I31P, V34P. The sequence of Pru p 3 construct 1 is:

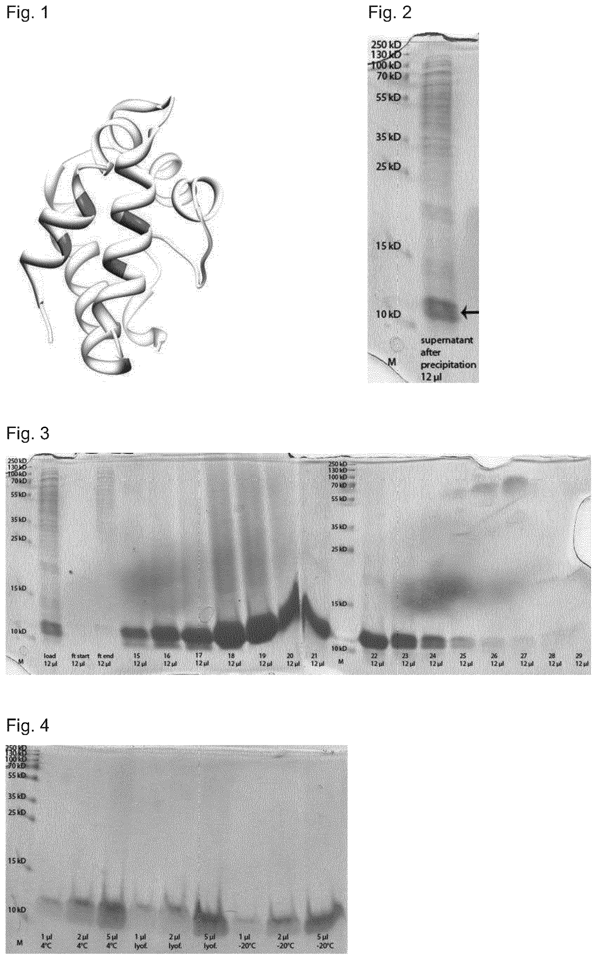

The protein has a calculated pl of 9.25 and a molecular weight of 9238.63 Da (calculated with http://expasy.org).
Pru p 3 construct 1 (see Fig. 1) was cloned into pHIS-parallel 2 (without tag) and expressed in *E.coli* Rosetta-gamiB, pLysS. Protein expression was induced by 0.4 mM IPTG and conducted at 16°C for 20 hours. The bacteria were harvested with centrifugation at 4.800 g and 4°C. Total protein expression (bacterial pellet resuspended in 1x SDS sample buffer) was checked on a 15% SDS-PAGE. The bacterial pellet was then dissolved in 1/5 culture volume 20 mM NaP buffer pH 4.48 with 8 M urea. The bacteria were broken up by freezing in liquid nitrogen and thawing at 37°C for three times. Furthermore the solution was ultra-turraxed for 10 min, sonicated for 10 min and then shaked at 220 rpm at RT o/n. Finally the solubilized proteins were separated from the cell debris by centrifugation for 20 min at 20.000 g and 4°C. Solubilization of Pru p 3 construct 1 was checked on a SDS-PAGE. Pru p 3 construct 1 (MW ≈ 10 Da) is solubilized in large parts in 20 mM NaP pH 4.48, 8 M urea. 10% acetic acid was slowly added to the solution under constant stirring until the pH was ≤ 4.00. Then the solution was centrifuged for 20 min at 20.000 g and 4°C and the supernatant containing Pru p 3 construct 1 was checked on SDS-PAGE (see Fig. 2). The precipitation of bacterial is an appropriate pre-purifications step. Several repetitions of this method demonstrated that Pru p 3 is not co-precipitating with other bacterial proteins.

The supernatant after the acidic precipitation was filtered through a 0.45 µm syringe filter and loaded onto a 5 ml HiTrap SP FF column with a flow rate of 1.5 - 2.0 ml/min, using an ÄKTA Prime Plus, GE Healthcare. The column was extensively washed with 20 mM NaP pH 4.00, 8 M Urea. The protein was refolded on the column by changing the buffer on the column to 20 mM NaP pH 4.00 without urea during a 400 ml gradient. The proteins which were bond to the column were eluted with a 60 ml gradient to 20 mM NaP pH 4.00 , 1MNaCl. The fractions were checked on gel electrophoresis and pure Pru p 3 construct 1 was pooled (see Fig. 3). Desalting of the buffer was accomplished using Amicon filters (3 kDa cut-off), resulting in a final buffer of 5mM NaP pH 4.48.

To test the storage stability of the protein, an aliquot was lyophilized in the SpeedVac and afterwards stored o/N at -20°C. Another aliquot was stored o/N at -20°C without lyophilisation. The rest of the solution containing the pure protein was stored at 4°C. The lyophilized aliquots were reconstituted in ddH₂O. The aliquot which was stored at -20°C is thawed, and then both aliquots were centrifuged for 20 min at 12.000 g. Since there was no pellet visible in the tube, it can be assumed that the protein is stable, both when stored at -20°C in solution and in the lyophilized state. To confirm this, different amounts were loaded onto a SDS-PAGE and compared to the solution stored at 4°C. On the gel equal amounts of protein, no matter under which conditions it was stored, are detectable (see Fig. 4). As a result Pru p 3 construct 1 is stable frozen in solution and frozen in a lyophilized state respectively. Therefore Pru p 3 construct 1 was lyophilized and stored at -20°C until further use.

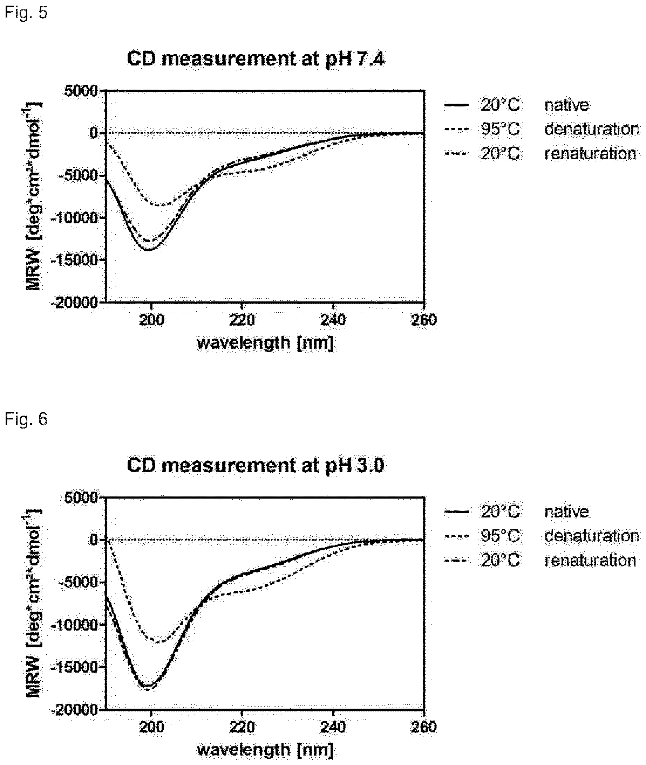

The secondary structure elements present in the protein were determined with circular dichroism spectroscopy. To see under which conditions the protein is more stable, the measurement was performed at two different pH levels (pH 3.0 and pH 7.4). At both pH values three spectra were taken. The first in the native conformation of Pru p 3 construct 1 at 20°C. The second was measured at 95°C, and the third is monitored upon cooling down to 20°C. Fig. 5 shows that minor parts of the alpha helical structure are still present in the protein, but the majority of the protein is in a random coil conformation. The spectrum measured at 95°C differs from the spectra taken at 20°C. As a result it can be assumed, that the protein is further unfolded at 95°C. The spectrum measured at 20°C after renaturation of the protein only slightly varies from the spectrum of the untreated protein. This means, that although the protein appears to be in a random coil conformation, there is a specific arrangement of amino acids that could be energetically preferred. The spectra taken at pH 3.0 are very similar to the spectra measured at pH 7.4 (Fig. 6). The only difference between the two pH levels is that at the low pH, the graphs of the untreated protein reach down to -18000 MRW, while at neutral pH they reach only -14000 MRW. At the lower pH, the difference between the initial spectrum and after heating are nearly identical, suggesting that the protein could be more stable at acidic conditions.

### Example 2: Mass spectrometry measurement of Pru p 3 construct 1

To characterize the protein more precisely, it was measured with ESI-Q-TOF mass spectrometry. Different measurements were performed to identify the primary sequence of the protein as well as modification.

### Intact MS measurements

### Untreated

The expected molecular weight of Pru p 3 construct 1 (including the N-terminal methionine) is 9238.63 Da (calculated with http://expasy.org). By taking the 4 disulfid bonds of the wild type protein into account the molecular weight is calculated to be 9230.63. In the spectrum 9 different major peaks were found. The first peak is at 9231.51 Da, and is therefore corresponding to the wild type protein with 4 intact disulfide bonds. The mass difference from one peak to another was always 43 Da. This mass difference indicates a modification of an amino acid. A possible explanation would be a carbamylation of the cysteines in the structure varying from no cysteine modified (9231.51 Da) to all 8 cysteines modified (9579.28 Da). Most frequently a carbamylation of 2, 3 or 4 cysteines was observed. To find out if the modification of Pru p 3 construct 1 is a carbamylation (MW = 43 Da), and to test if the reaction is reversible, further MS measurements were performed and acknowledged using tryptic peptides Calbiochem "ProteoExtract All-In-One Trypsin Digestion Kit".The MS measurements confirmed the primary sequenc of Pru p 3 construct 1. Additionally, the MS showed that the cysteines in the primary structure are carbamylated in a statistical manner. This means that potentially all cysteines can be modified with a carbamyl group but the distribution of the modification is not following an obvious pattern. The carbamylation is reversible by reduction and alkylation although a small part of the carbamylations is resistant to reduction or alternatively the carbamyl group is splitted off and then instantly is reacting again with the protein.

### Example 3: IgE binding properties of Pru p 3 construct 1 with ELISA ELISA protocol

| | |
|---|---|
| coating | 4 µg/ml protein - 50 µl per well |
| | 4°C over night |
| | *Buffer: 1xPBS* |
| wash | 2 x 200 µl per well |
| | *Buffer: 1xTBS, 0.05% tween* |
| blocking #1 | 1 h at RT - 200 µl per well |
| | *Buffer: 1xTBS, 0.5% BSA, 0.05% tween* |
| wash | 2 x 200 µl per well |
| | *Buffer: 1xTBS, 0.05% tween* |
| blocking #2 (cysteine blocking) | 1 h at RT - 100 µl per well |
| | *Buffer: 1xTBS, 0.05% tween* +*100 mM L-cysteine* |
| wash | 10 x 200 µl per well |
| | *Buffer: 1xTBS, 0.05% tween* |
| serum | 1:4 - 50µl per well |
| | 4°C over night |
| | *Buffer: 1xTBS, 0.5% BSA, 0.05% tween* |
| wash | 4 x 100 µl per well |
| | *Buffer: 1xTBS, 0.05% tween* |
| 2nd antibody | 1:2000 - 50 µl per well |
| | 1h 37°C and 1h 4°C |
| | *Buffer: 1xTBS, 0.5% BSA, 0.05% tween* |
| wash | 4 x 100 µl per well |
| | *Buffer: 1xTBS, 0.05% tween* |
| substrate | 100 µl per well 10 mM PNPP |
| | *Buffer: AP-detection buffer for ELISA* |

An ELISA with the patients' sera (n=10) was performed following the given ELISA protocol, including a pre-incubation step with cysteine since unspecific reactions with the 2^{nd} antibody were observed. After the remaining free cysteine was washed away, the patient sera (1:4 diluted) were applied. The following data were obtained by measuring the absorbance (405/495 nm) after 30 min of incubation with the substrate (Table 1).

**Table 1**

| patient number | OD_{405/492nm} for rPru p 3 WT solid-phase coates wells [mAu] | OD_{405/492nm} for Pru p 3 construct 1 solid-phase coates wells [mAu] | remaining reactivity [%] |
|---|---|---|---|
| 48567 | 3.078 | 0.737 | 23.93 |
| 48568 | 2.915 | 0 | 0.00 |
| 48569 | 1.107 | 0.171 | 15.41 |
| 48570 | 0.247 | 0.116 | 46.75 |
| 48572 | 0.805 | 0 | 0.00 |
| 48573 | 3.773 | 0 | 0.00 |
| 48574 | 0.130 | 0 | 0.00 |
| 48576 | 0.434 | 0 | 0.00 |
| 48577 | 1.069 | 0.195 | 18.20 |
| 48579 | 0.705 | 0.0127 | 1.80 |

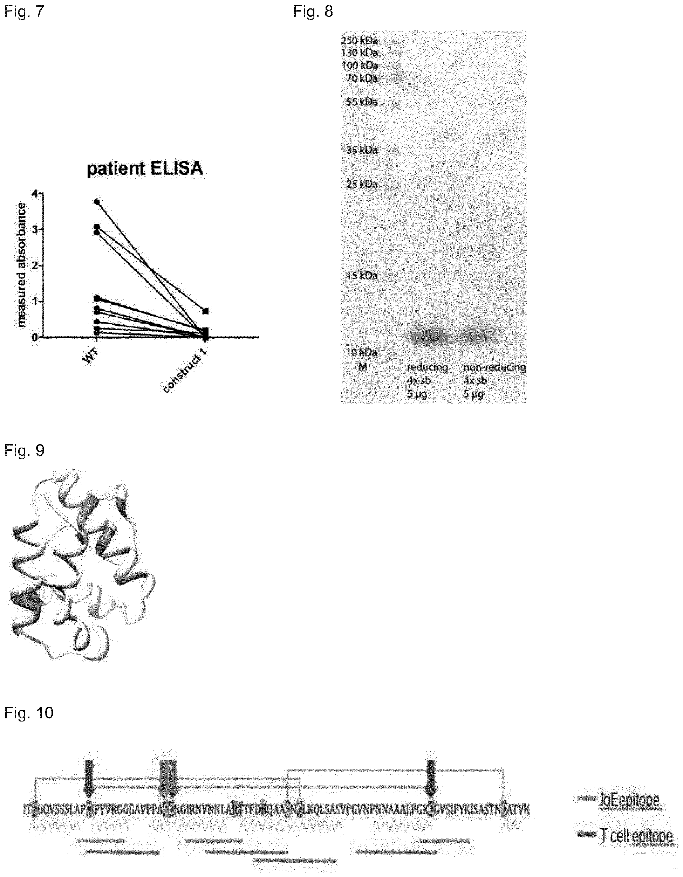

The data show that Pru p 3 construct 1 has a reduced IgE binding capacity compared to the WT molecule (Fig. 7). The mean reduction of all patients results is 89.39%. The majority of patients showed a completely abrogated IgE reactivity to Pru p 3 construct 1.

To confirm that Pru p 3 construct 1 is not forming disulfide bond mediated multimers, gel electrophoresis was performed under reducing and non reducing conditions. Therefore 2.5 µl (corresponding to 5 µg protein) were loaded onto the gel. For the reducing conditions a 4x sample buffer containing β-mercaptoethanol was added to the protein solution. For the non-reducing conditions a 4x sample buffer without β-mercaptoethanol was used. After dying the proteins with Coomassie Brilliant Blue G-250; BioRad and destaining the gel the following picture was observed. The SDS-PAGE (Fig. 8) shows a single band at around 11 kDa under reducing and also under non-reducing conditions. As a result it can be said, that Pru p 3 construct 1 is monomeric and there are no dimers or oligomeres resulting from intermolecular disulfide bond formations.

### Example 4: Pru p 3 construct 4

In this construct two amino acids where exchanged. Two cysteines (C27 and C28) involved in two different disulfide bonds (C27-C13 and C28-C73) were replaced by alanines, which cause a disruption of the typically observed disulfide bond pattern of lipid transfer proteins. Based on the proximity between the residues C13 and C73 a possible formation of a new disulfide bond might occur (Fig. 9).

In Fig. 10 the sequence of Pru p 3 is depicted, the IgE and the T-cell epitopes are marked. The red arrows flag the 2 cysteines which were mutated into alanines. Highlighted with the blue arrows are the cysteines that could potentially form a new disulfide bond.

### Pru p 3 construct 4 expression and IMPACT system

### Overview expression conditions

The IMPACT *(Intein Mediated Purification with an Affinity Chitin-binding Tag)* system (New England BioLabs) is a protein purification system which uses the self-cleavage activity of certain protein splicing elements (inteins) to separate the target protein (Pru p 3 const. 4) from the affinity tag (chitin binding domain).

The intein fusion protein contains a chitin binding domain (CBD) for the affinity purification on a chitin bead column and the target protein (Pru p 3 construct 4). Induction of on-column cleavage is done with a thiol reagent (for example DTT, ß-mercaptoethanol or cysteine) which leads to a release of the target protein. After elution the chitin-binding parts of the protein are remaining on the column and the target protein will elute in the first few fractions. For Pru p 3 construct 4 an activation/cleavage time of 20 h at RT is found to be optimal. But still only a part of the column bound fusions-proteins will be cleaved. This is why repeated activation steps were conducted.

The bacteria (Rosetta gami B, pLysS with the Pru p 3 construct 4 in TXB1) were plated on LB ampicillin plates and incubated overnight at 37°C (1 plate for 1l liquid culture). The next day the plated bacteria were washed into prepared liquid LB ampicillin medium. A starting OD of 0.1 is ideal for growth and expression. 1l shaking flasks with 400 - 500 ml culture are recommended, because the expression under this condition is best. The culture was grown to an OD of about 0.5 - 0.6 and put onto 16°C, after the cooling the OD of was checked again and at an OD of 0.7 the culture was induced with 0.4 mM IPTG. Expression was done for 18-20h overnight at 16°C with 200 rpm. After the end of expression the bacteria were centrifuged with 4500 g at 4°C, the supernatant was discard and the pellet dissolved in cell lysis buffer [20 mM TRIS/HCI (ph 8.5), 500 ml NaCl, 2 M urea], for 1l of culture 100 ml buffer were used. The bacteria were frozen with liquid nitrogen and thawed at 37°C. This process was repeated 3 times to break up the bacteria. The bacterial breakup was pooled and homogenized with Ultra-Turrax for 5 min until the solution was completely foamy. The homogenizate was sonicated for 10 min on ice. After this DNAse was added (3 µg/ml) and the solution was incubated for 40 min with 50 rpm. The solution was centrifuged with 12.000 g for 20 min at 4°C. The supernatant was stored at 4°C until the loading onto the column.

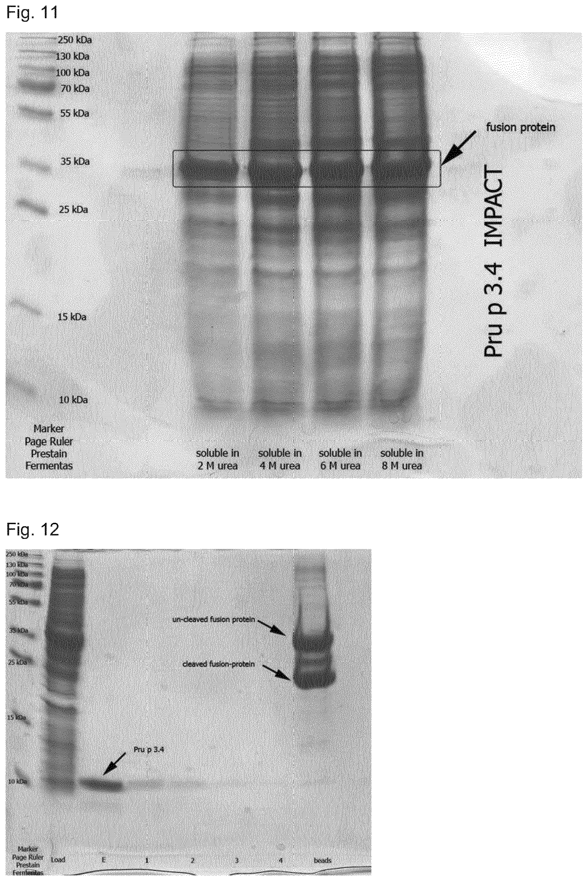

The fusion-protein is only soluble in a buffer containing urea. The IMPACT system allows an addition of urea up to 4 M. With the addition of 2 M urea, an adequate solubilization of the fusion-protein is achievable and was used for further experiments (Fig. 11).

### Purification

A 20 ml column with 10 ml of chitin-beads was packed, the beads were stored in ethanol so they had to be washed with 20 bed volumes of dH₂O at first and then equilibrated with 20 bed volumes of column buffer *[20 mM TRISlHCl (ph 8.5), 500 ml NaCl, 2 M ureal.* The protein solution was loaded onto the column with a flow rate of about 1 ml/min. The capacity of the column (10 ml bed volume) was about 150-200 ml protein solution this is equal to 1.5l - 2l expression. The unbound fractions were checked on a SDS-PAGE to analyze the binding capacity of the column. The column was washed with 30-40 bed volumes of column buffer, a high flow rate (3-4 ml/min) was chosen because the binding between the chitin-beads and the chitin-binding domain of the protein is very strong and so the bacterial proteins were easier washed off the column.

The cleavage of Pru p 3 from the fusion protein is activated with a thiol reagent (for example L-cysteine). After the washing step the column was flushed with 30-50 ml fresh prepared cleavage buffer containing 50 mM L-cysteine. During the cleavage progress the column was stored at RT for 16-20 h before the elution of the target protein. The protein was eluted with column buffer and first 10 ml were collected. Additionally the following elution was collected in 1.5 ml fractions, to avoid unnecessary dilution of the protein.

The elution and the 1.5 ml fraction were analyzed on a SDS-PAGE. A sample of the column beads was taken and also checked on the SDS-PAGE to analyze the cleavage efficiency. As visible in Fig. 12, the elution (E) and the fractions (1, 2 and 3) contained the target protein *(Pru p 3 construct 4).* The tested sample of the chitin beads showed that only a part of the fusion-protein was cleaved which indicates that a second (or third) activation with cysteine is mandatory. For Pru p 3 construct 4, two additional activations were performed which resulted in the majority of fusion protein to be cleaved.

### Buffer exchange and concentration

To change the buffer to a neutral, low salt buffer, a buffer exchange with Amicon filter (3.000 Da cutoff) was performed. The Amicon filter was first equilibrated with 15 ml dH₂O and centrifuged for 10 min with 3.500 g. After the equilibration step the remaining dH₂O was discarded. 5 ml from the Pru p 3 construct 4 elution were mixed with 10 ml of 5 mM NaP pH 7.4 and filled into the Amicon filter. The tube was then centrifuged for 20 min with 3.500 g. This step was repeated 6 times and after the last dilution the protein should be present in 5 mM NaP pH 7.4.

### Example 5: Characterization of Pru p 3 construct 4

To investigate the secondary structure elements of the purified Pru p 3 construct Circular dichroism (CD) analysis was performed following the procedure given above.

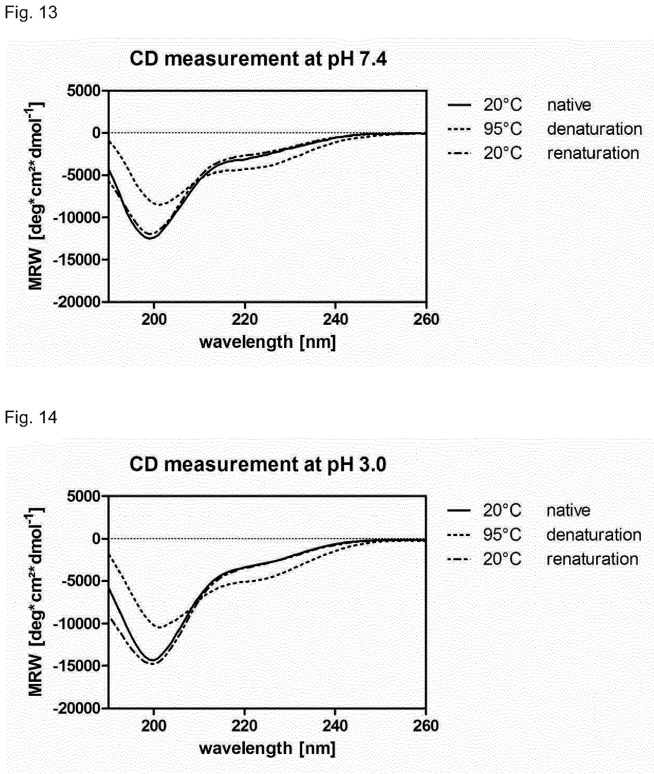

The CD curves show that the protein is not completely unfolded because there was still a change in the CD-spectrum after denaturation (Fig 13 and 14). The protein furthermore established a similar curve upon renaturation, thus it can be assumed that the molecule contains at least some defined secondary structure elements. Furthermore a difference between the pH 7.4 and pH 3.0 spectrum is identifiable. Under pH 3.0 conditions the MRW (deg*cm^{2*}dmol⁻¹) value was at - 15.000 which is lower than in the pH 7.4 spectrum where the MRW was at -12.500 (native and renatured measurement; wavelength 200 nm). It can be assumed that the protein is more stable under the pH 3.0 conditions, which is typical for some LTPs.

### Example 6: Mass spectrometry (MS) Analysis of Pru p 3 construct 4

All samples were measured with an ESI Q-TOF mass spectrometer analogous to measurements given above. The DNA sequence was translated into amino acids. Amino acid sequence of Pru p 3 construct 4 (SEQ ID NO:10)

At the C-terminus of the protein, 8 additional amino acids are present (CGRATTVK), also an extra cysteine (C) is usually placed at the C-terminus after the intein cleavage due to the chemical process. The start methione at position 1 was thought to be not present in the processed protein, which was confirmed with the tryptic digestion measurement. The mass spectrum for the intact untreated Pru p 3 construct 4 showed a Gaussian distribution of different values. Between these values a mass difference of 119-120 was observed. This indicates a cysteinylation of the cysteine residues with free cysteines from the activation step of the intein cleavage.

The mass for the sequence was calculated with **PeptidMass (expasy.org)** which resulted in a mass of 10070.6275 Da. To this mass 119.1 Da for every possible cysteinylation was added. Also possible disulfide bonds were considered. The calculated values could be assigned to the experimental values (Table 2).

**Table 2**

| **calculated values** | | | | **measured peaks** | **difference** |
|---|---|---|---|---|---|
| number of cysteinylations | mass | -H atoms for disulfide bonds | corrected mass | | |
| + 0 cysteinylation | 10070.6275 | -6 | 10064.6275 | 10065.9424 | 1.3149 |
| + 1 cysteinylation | 10189.7275 | -4 | 10185.7275 | 10186.1338 | 0.4063 |
| + 2 cysteinylation | 10308.8275 | -4 | 10304.8275 | 10306.0664 | 1.2389 |
| + 3 cysteinylation | 10427.9275 | -2 | 10425.9275 | 10426.4277 | 0.5002 |
| + 4 cysteinylation | 10547.0275 | -2 | 10545.0275 | 10546.0361 | 1.0086 |
| + 5 cysteinylation | 10666.1275 | 0 | 10666.1275 | 10666.3662 | 0.2387 |
| + 6 cysteinylation | 10785.2275 | 0 | 10785.2275 | 10785.9482 | 0.7207 |

Upon reduction and alkylation of the sample, Pru p 3 construct 4 demonstrates a mass of 10529.03 which is in accordance with the theoretical value of 10527.04.

In solution digestion with trypsin was performed and the samples were measured. The results showed that the majority of the possible fragments were found (sequence coverage 84.78%).In accordance with results from the intact mass measurement, the cysteine residues of the protein were partly cysteinylated.

### Example 7: Pru p 3 construct 4 ELISA with patients sera

Sera of 10 Pru p 3 allergic patients were tested in ELISA as described above. All 10 patients reacted to the WT Pru p 3, the reaction to Pru p 3 construct 4 was very low, and strongly reduced in all 10 patients.

**Table 3**

| **#** | **Patiens ID** | **measured values** | | **reduction in %** |
|---|---|---|---|---|
| | | WT | Pru p 3 c4 v1 | Remaining reactivity (%) |
| 1 | 48567 | 3.078 | 0.000 | 0 |
| 2 | 48568 | 2.915 | 0.000 | 0 |
| 3 | 48569 | 1.107 | 0.000 | 0 |
| 4 | 48570 | 0.247 | 0.000 | 0.1 |
| 5 | 48572 | 0.805 | 0.000 | 0 |
| 6 | 48573 | 3.773 | 0.064 | 1.7 |
| 7 | 48574 | 0.130 | 0.002 | 1.8 |
| 8 | 48576 | 0.434 | 0.000 | 0 |
| 9 | 48577 | 1.069 | 0.005 | 0.5 |
| 10 | 48579 | 0.705 | 0.070 | 10 |

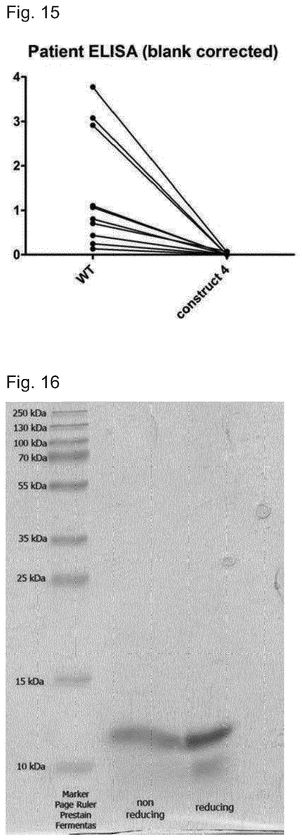

In Fig. 15 the average values for Pru p 3 construct 4 and the WT are given. The reactivity against the WT Pru p 3 and Pru p 3 construct 4 was correlated and is showed in the following graph. As shown in the figures, Pru p 3 construct 4 showed a strongly reduced IgE reactivity. For the majority of patients the IgE reactivity was in the range of the buffer control.

The mean reduction in IgE reactivity of all patients is 98.58%.

### SDS-PAGE reducing / non-reducing

Pru p 4 construct 4 was tested for the presence of disulfide bond related multimers using gel electrophoresis as described above. Pru p 3 construct 4 is migrating as single band at approx. 12 kDa in reducing and non-reducing conditions (Fig 16).

## Claims

1. A recombinant Pru p 3 mimick having the sequence of SEQ ID NO:1, which is modified by substituting at least one amino acid and which is capable of inducing IgG antibodies that are cross-reactive to the Pru p 3 wild-type allergen, and where reactivity to specific IgE antibodies is reduced and wherein at least one substitution is at a position outside the T-cell epitopes and wherein the cysteines at positions 48 and 50 remain unsubstituted.

2. The Pru p 3 mimick according to claim 1, where the capacity of inducing IgG antibodies is similar to the wild-type Pru p 3 allergen.

3. The Pru p 3 mimick according to claim 1 or 2, which has in average less than 10 % reactivity to specific IgE antibodies compared to the wild-type Pru p 3 allergen.

4. The Pru p 3 mimick according to any one of claims 1 to 3, wherein the at least one substituted amino acid is proline at a position outside the T-cell epitopes.

5. The Pru p 3 mimick according to claim 4, wherein said at least one substituted amino acid is at least one proline at a position outside the SEQ ID NO:2, SEQ ID NO:3, SEQ ID NO:4 and SEQ ID NO:5.

6. The Pru p 3 mimick according to any one of claims 1 to 5, wherein at least one and up to 6 amino acids are substituted at any position outside the T-cell epitopes.

7. The Pru p 3 mimick according to claim 6, wherein the cysteines at positions 3, 48, 50 and/or 87 remain unsubstituted.

8. The Pru p 3 mimick according to any one of claims 1 to 7, wherein the Pru p 3 mimick has an alpha helical secondary structure stabilized by at least the distal disulfide bonds.

9. The Pru p 3 mimick, wherein the amino sequence is described by the general formula:
(M)ₙIT(X1)GQ(X2)SSS(X3)AP(X4)IPYVRGGGAVPPA(X5)(X6)NG(X7)RN(X8)N NLARTTPDRQAA(X9)N(X10)LKQLSASVPGVNPNNAAALPGK(X11)GVSIPYKI SASTN(X12)ATVK
wherein X1 is any amino acid residue, preferably it is C,
X2 is a V or P residue,
X3 is a L or P residue,
X4 is any amino acid residue, preferably it is C,
X5 is a C, P or A residue, preferably it is A,
X6 is a C, P or A residue, preferably it is A,
X7 is a I or P residue,
X8 is a V or P residue,
X9 is any amino acid residue, preferably it is C,
X10 is any amino acid residue, preferably it is C,
X11 is any amino acid residue, preferably it is C,
X12 is any amino acid residue, preferably it is C,
n can be either 0 or 1, wherein at least one of positions X1, X2, X3, X4, X5, X6,X7, X8, X9, X10, X11 or X12 are modified (SEQ ID No. 6), wherein at least one substitution is at a position outside the T-cell epitopes and wherein the cysteines at positions 48 and 50 remain unsubstituted.

10. A method for producing a recombinant Pru p 3 mimick according to any one of claims 1 to 10, using recombinant technique, wherein a nucleotide sequence encoding the Pru p 3 mimick is constructed in a vector and expressed in a host cell.

11. An isolated nucleic acid molecule encoding for a Pru p 3 mimick protein according to any one of claims 1 to 9.

12. A Pru p 3 mimick according to any one of claims 1 to 9 for use as a medicament.

13. The use according to claim 12, wherein said Pru p 3 mimick induces specific IgG antibodies capable of recognizing also the corresponding wild type allergen, but reduces capability of induction of specific IgE antibodies.

14. A pharmaceutical composition comprising the Pru p 3 mimick according to any one of claim 1 to 9, in combination with pharmaceutically acceptable excipients for the prophylactic or therapeutic treatment of allergic diseases.

15. A vaccine comprising a Pru p 3 mimick according to any one of claims 1 to 9, preferably comprising further at least one pharmaceutical acceptable adjuvant, excipient and/or carrier.

## Patentansprüche

1. Rekombinanter Pru-p3-Nachahmer mit der Sequenz von SEQ-ID-Nr. 1, der durch Substituieren mindestens einer Aminosäure modifiziert ist und der fähig ist, IgG-Antikörper zu induzieren, die dem Pru-p3-Wildtyp-Allergen gegenüber kreuzreaktiv sind, und wobei die Reaktivität gegenüber spezifischen IgE-Antikörpern reduziert ist und wobei mindestens eine Substitution an einer Position außerhalb der T-Zell-Epitope ist und wobei die Cysteine an Position 48 und 50 unsubstituiert bleiben.

2. Pru-p3-Nachahmer nach Anspruch 1, wobei die Fähigkeit zum Induzieren von IgG-Antikörpern ähnlich wie beim Wildtyp-Pru-p3-Allergen ist.

3. Pru-p3-Nachahmer nach Anspruch 1 oder 2, der im Vergleich zu dem Wildtyp-Pru-p3-Allergen im Durchschnitt weniger als 10 % Reaktivität gegenüber spezifischen IgE-Antikörpern aufweist.

4. Pru-p3-Nachahmer nach einem von Anspruch 1 bis 3, wobei die mindestens eine substituierte Aminosäure Prolin an einer Position außerhalb der T-Zell-Epitope ist.

5. Pru-p3-Nachahmer nach Anspruch 4, wobei die mindestens eine substituierte Aminosäure mindestens ein Prolin an einer Position außerhalb der SEQ-ID-Nr. 2, SEQ-ID-Nr. 3, SEQ-ID-Nr. 4 und SEQ-ID-Nr. 5 ist.

6. Pru-p3-Nachahmer nach einem von Anspruch 1 bis 5, wobei mindestens eine und bis zu 6 Aminosäuren an einer beliebigen Position außerhalb der T-Zell-Epitope substituiert sind.

7. Pru-p3-Nachahmer nach Anspruch 6, wobei die Cysteine an Position 3, 48, 50 und/oder 87 unsubstituiert bleiben.

8. Pru-p3-Nachahmer nach einem von Anspruch 1 bis 7, wobei der Pru-p3-Nachahmer eine alphahelikale Sekundärstruktur aufweist, welche mindestens durch die distalen Disulfidbindungen stabilisiert ist.

9. Pru-p3-Nachahmer, wobei die Aminosequenz durch die folgende allgemeine Formel beschrieben ist:
wobei X ein beliebiger Aminosäurerest ist, bevorzugt ist es C,
X2 ein V- oder P-Rest ist,
X3 ein L- oder P-Rest ist,
X4 ein beliebiger Aminosäurerest ist, bevorzugt ist es C,
X5 ein C-, P- oder A-Rest ist, bevorzugt ist es A,
X6 ein C-, P- oder A-Rest ist, bevorzugt ist es A,
X7 ein I- oder P-Rest ist,
X8 ein V- oder P-Rest ist,
X9 ein beliebiger Aminosäurerest ist, bevorzugt ist es C,
X10 ein beliebiger Aminosäurerest ist, bevorzugt ist es C,
X11 ein beliebiger Aminosäurerest ist, bevorzugt ist es C,
X12 ein beliebiger Aminosäurerest ist, bevorzugt ist es C,
n entweder 0 oder 1 sein kann und wobei mindestens eine von Position X1, X2, X3, X4, X5, X6, X7, X8, X9, X10, X11 und X12 modifiziert ist (SEQ-ID-Nr. 6), wobei mindestens eine Substitution an einer Position außerhalb der T-Zell-Epitope ist und wobei die Cysteine an Position 48 und 50 unsubstituiert bleiben.

10. Verfahren zum Herstellen eines rekombinanten Pru-p3-Nachahmers nach einem von Anspruch 1 bis 10 unter Benutzen rekombinanter Technik, wobei eine Nukleotidsequenz, die den Pru-p3-Nachahmer codiert, in einem Vektor konstruiert wird und in einer Wirtszelle exprimiert wird.

11. Isoliertes Nukleinsäuremolekül, das für ein Pru-p3-Nachahmerprotein nach einem von Anspruch 1 bis 9 codiert.

12. Pru-p3-Nachahmer nach einem von Anspruch 1 bis 9 zur Benutzung als ein Medikament.

13. Benutzung nach Anspruch 12, wobei der Pru-p3-Nachahmer spezifische IgG-Antikörper induziert, die fähig sind, auch das entsprechende Wildtyp-Allergen zu erkennen, jedoch die Fähigkeit zur Induktion von spezifischen IgE-Antikörpern reduziert.

14. Pharmazeutische Zusammensetzung, umfassend den Pru-p3-Nachahmer nach einem von Anspruch 1 bis 9, in Kombination mit pharmazeutisch akzeptablen Hilfsstoffen zur prophylaktischen oder therapeutischen Behandlung von allergischen Erkrankungen.

15. Impfstoff, umfassend einen Pru-p3-Nachahmer nach einem von Anspruch 1 bis 9, vorzugsweise ferner umfassend mindestens einen pharmazeutisch akzeptablen Wirkungsverstärker, Hilfsstoff und/oder Träger.

## Revendications

1. Mimique de Pru p 3 recombinante ayant la séquence de SÉQ ID n° : 1, qui est modifiée en substituant au moins un acide aminé et qui est capable d'induire des anticorps IgG qui ont une réactivité croisée vis-à-vis de l'allergène Pru p 3 de type sauvage, et où la réactivité vis-à-vis des anticorps IgE spécifiques est réduite et dans laquelle au moins une substitution est au niveau d'une position en dehors des épitopes T et dans laquelle les cystéines au niveau des positions 48 et 50 restent non substituées.

2. Mimique de Pru p 3 selon la revendication 1, où la capacité d'induire des anticorps IgG est similaire à l'allergène Pru p 3 de type sauvage.

3. Mimique de Pru p 3 selon la revendication 1 ou 2, qui a en moyenne moins de 10 % de réactivité vis-à-vis des anticorps IgE spécifiques par rapport à l'allergène Pru p 3 de type sauvage.

4. Mimique de Pru p 3 selon l'une quelconque des revendications 1 à 3, dans laquelle l'au moins un acide aminé substitué est la proline au niveau d'une position en dehors des épitopes T.

5. Mimique de Pru p 3 selon la revendication 4, dans laquelle ledit au moins un acide aminé substitué est une proline au niveau d'une position en dehors de SÉQ ID n° : 2, SÉQ ID n° : 3, SÉQ ID n° : 4 et SÉQ ID n° : 5.

6. Mimique de Pru p 3 selon l'une quelconque des revendications 1 à 5, dans laquelle au moins un et jusqu'à 6 acides aminés sont substitués au niveau d'une quelconque position en dehors des épitopes T.

7. Mimique de Pru p 3 selon la revendication 6, dans laquelle les cystéines au niveau des positions 3, 48, 50 et/ou 87 restent non substituées.

8. Mimique de Pru p 3 selon l'une quelconque des revendications 1 à 7, laquelle mimique de Pru p 3 a une structure secondaire hélicoïdale alpha stabilisée par au moins les liaisons disulfures distales.

9. Mimique de Pru p 3, dans laquelle la séquence d'acides aminés est décrite par la formule générale :
dans laquelle X1 est un quelconque résidu d'acide aminé, de préférence est C,
X2 est un résidu de V ou P,
X3 est un résidu de L ou P,
X4 est un quelconque résidu d'acide aminé, de préférence est C,
X5 est un résidu de C, P ou A, de préférence est A,
X6 est un résidu de C, P ou A, de préférence est A,
X7 est un résidu de I ou P,
X8 est un résidu de V ou P,
X9 est un quelconque résidu d'acide aminé, de préférence est C,
X10 est un quelconque résidu d'acide aminé, de préférence est C,
X11 est un quelconque résidu d'acide aminé, de préférence est C,
X12 est un quelconque résidu d'acide aminé, de préférence est C,
n peut valoir soit 0 soit 1, dans laquelle au moins une des positions X1, X2, X3, X4, X5, X6, X7, X8, X9, X10, X11 ou X12 est modifiée (SÉQ ID n° : 6), dans laquelle au moins une substitution est une position en dehors des épitopes T et dans laquelle les cystéines au niveau des positions 48 et 50 restent non substituées.

10. Procédé de production d'une mimique de Pru p 3 recombinante selon l'une quelconque des revendications 1 à 10, à l'aide d'une technique de recombinaison, dans lequel une séquence nucléotidique codant pour la mimique de Pru p 3 est construite dans un vecteur et exprimée dans une cellule hôte.

11. Molécule d'acide nucléique isolée codant pour une protéine mimique de Pru p 3 selon l'une quelconque des revendications 1 à 9.

12. Mimique de Pru p 3 selon l'une quelconque des revendications 1 à 9 pour une utilisation en tant que médicament.

13. Utilisation selon la revendication 12, dans laquelle ladite mimique de Pru p 3 induit des anticorps IgG spécifiques capables de reconnaître également l'allergène de type sauvage, mais réduit la capacité d'induire des anticorps IgE spécifiques.

14. Composition pharmaceutique comprenant la mimique de Pru p 3 selon l'une quelconque des revendications 1 à 9, en combinaison avec des excipients pharmaceutiquement acceptables pour le traitement prophylactique ou thérapeutique de maladies allergiques.

15. Vaccin comprenant une mimique de Pru p 3 selon l'une quelconque des revendications 1 à 9, comprenant de préférence également au moins un adjuvant, excipient et/ou support pharmaceutiquement acceptables.
